# EUROPÄISCHE PATENTSCHRIFT

(11) **EP 2 049 254 B1**
(45) Veröffentlichungstag und Bekanntmachung des Hinweises auf die Patenterteilung: **15.12.2010**
(21) Anmeldenummer: 07787881.7
(22) Anmeldetag: 25.07.2007
(51) Int. Cl.: B01J 23/46, B01J 38/04, C07C 67/303, C07C 51/36

(54) **VERFAHREN ZUR REGENERIERUNG VON RUTHENIUMKATALYSATOREN FÜR DIE KERNHYDRIERUNG VON PHTHALATEN**
PROCESS FOR REGENERATING RUTHENIUM CATALYSTS FOR THE RING HYDROGENATION OF PHTHALATES
PROCÉDÉ DE RÉGÉNÉRATION DE CATALYSEURS DE RUTHÉNIUM UTILISÉS POUR L'HYDROGÉNATION DU NOYEAU DE PHTALATES

(30) Priorität: 31.07.2006 EP 06118205
(43) Veröffentlichungstag der Anmeldung: 22.04.2009
(73) Patentinhaber: BASF SE, 67056 Ludwigshafen (DE)
(72) Erfinder: HENKELMANN, Jochem, 68165 Mannheim (DE); BECKER, Michael, 77654 Offenburg (DE); RICHTER, Felix, 67069 Ludwigshafen (DE); SCHÄFER, Thomas, 68165 Mannheim (DE)
(86) Internationale Anmeldenummer: PCT/EP2007/057651
(87) Internationale Veröffentlichungsnummer: WO 2008/015135

(56) Entgegenhaltungen:
- EP-A1- 0 913 194
- DE-A1- 10 128 242
- JP-A- 1 159 059
- JP-A- 3 068 453
- JP-A- 2000 051 701

## Beschreibung

Die vorliegende Erfindung betrifft ein integriertes Verfahren zur Hydrierung von Phthalaten zu den entsprechenden Cyclohexandicarbonsäurederivaten mit Regenerierung des Katalysators.

Ein Verfahren zur Herstellung von ausgewählten Cyclohexan-1,3- und Cyclohexan-1,4-Dicarbonsäuren wird in der WO 00/78704 offenbart. Diese Verbindungen eignen sich hervorragend als Weichmacher. Insbesondere zu erwähnen ist Cyclohexan-1,4-dicarbonsäurediisononylester.

Ein besonders geeigneter Katalysator, der bei der Hydrierung aromatischer Verbindungen eingesetzt werden kann, wird in der DE 196 24 485 A1 offenbart. Der Katalysator umfasst als Aktivmetall Ruthenium alleine oder zusammen mit mindestens einem Metall der I., VII., oder VIII. Nebengruppe des Periodensystems (CAS-Version) in einer Menge von 0,01 bis 30 Gew.-% bezogen auf das Gesamtgewicht des Katalysators und ist auf einen Träger aufgebracht. 10 bis 50% des Porenvolumens des Trägers werden von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet, wobei sich die Summe der Porenvolumina zu 100% addiert. Als Träger werden Aktivkohle, Siliziumcarbid, Aluminiumoxid, Siliziumdioxid, Titandioxid, Zirkoniumdioxid, Magnesiumdioxid, Zinkoxid oder ein Gemisch aus zwei oder mehr davon eingesetzt.

Weitere besonders geeignete Katalysatoren zur Hydrierung aromatischer Verbindungen werden in der EP-A 1 169 285 offenbart. Der Katalysator umfasst in einer Ausführungsform (Katalysator 1) mindestens ein Metall der VIII. Nebengruppe des Periodensystems (CAS-Version), aufgebracht auf einen Träger, wobei der Träger Makroporen aufweist und der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfasst, wobei der Träger einen mittleren Porendurchmesser von mindestens 50 nm und eine BET-Oberfläche von höchstens 30 m²/g aufweist und die Menge des Aktivmetalls 0,01 bis 30 Gew.- %, bezogen auf das Gesamtgewicht des Katalysator, beträgt. In einer weiteren Ausführungsform (Katalysator 2) umfasst der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei die Summe der Anteile der Porenvolumina zu 100% beträgt. Als Träger werden Aktivkohle, Siliziumcarbid, Aluminiumoxid, Siliziumdioxid, Titandioxid, Zirkoniumdioxid, Magnesiumdioxid, Zinkoxid oder ein Gemisch aus zwei oder mehr davon eingesetzt, vorzugsweise Aluminiumoxid.

Schließlich ist ein weiterer besonders geeigneter Katalysator in der Anmeldung DE 102 005 029 200 offenbart. Es handelt sich um einen Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial, **dadurch gekennzeichnet, dass** die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen, ermittelt mittels SEM-EPMA (EDXS).

Der Erhalt der Katalysatoraktivität über einen möglichst langen Zeitraum ist für industrielle Prozesse von großer wirtschaftlicher Bedeutung.

Üblicherweise wird ein Nachlassen der katalytischen Aktivität durch verschiedene physikalische und chemische Effekte auf den Katalysator hervorgerufen, zum Beispiel durch Blockieren der katalytisch aktiven Zentren beziehungsweise durch Verlust katalytisch aktiver Zentren durch thermische, mechanische oder chemische Prozesse. Beispielsweise kann eine Katalysatordeaktivierung oder allgemein Alterung durch Sintern der katalytisch aktiven Zentren, durch Verlust von (Edel-)Metall, durch Ablagerungen oder durch Vergiftung der aktiven Zentren verursacht werden. Die Mechanismen der Alterung/Deaktivierung sind vielfältig.

Herkömmlicherweise muss der deaktivierte Katalysator zur Regenerierung aus dem Reaktor entfernt werden. Danach liegt der Reaktor still, oder der Betrieb wird nach Einbau eines anderen Katalysators wieder aufgenommen. Dies führt in jedem Fall zu signifikanten Kosten. In den US-Patenten US 3,851,004 und US 2,757,128 sind Verfahren zur Hydrierung von u. a. Olefinen in Kohlenwasserstoff-Ausgangsmaterialien, sowie die Regenerierung der Katalysatoren mittels Wasserstoff offenbart.

Die DE 196 34 880 C2 offenbart ein Verfahren zum gleichzeitigen selektiven Hydrieren von Diolefinen und Nitrilen aus einem Kohlenwasserstoff-Ausgangsmaterial. Bei diesem Verfahren wird der Katalysator, nachdem dessen Diolefin-Hydrierungsaktivität auf weniger als 50% der anfänglichen Aktivität gesunken ist, mit einem Inertgas zum Entfernen von Spuren des Kohlenwasserstoffs aus dem Katalysator und zum Schaffen eines gespülten Katalysators durchspült und in einem anschließenden Regenerierungsschritt mit Wasserstoff durchspült. Hierbei wird ein regenerierter Katalysator erzeugt, dessen Diolefin-Hydrierungsaktivität wieder mindestens 80% des anfänglichen Wertes aufweist.

Die EP 0 913 194 A1 beschreibt ein Verfahren zur Regenerierung eines Rutheniumkatalysators, der bei der Hydrierung ungesättigter organischer Verbindungen eingesetzt wird und dabei an Aktivität verliert. Zur Regenerierung wird der deaktivierte Katalysator in flüssiger Phase, beispielsweise als Suspension mit Sauerstoff in Kontakt gebracht und in einem weiteren Schritt bei einem Wasserstoffpartialdruck unterhalb des bei der Hydrierung herrschenden Wasserstoffpartialdruck und bei einer Temperatur, die höchstens 50°C unterhalb der Hydriertemperatur liegt, gehalten.

Bei der Hydrierung von Phthalaten unter Verwendung der beschriebenen Rutheniumkatalysatoren ist ebenfalls eine Desaktivierung zu beobachten, die bis jetzt nicht auf einfachem Weg beseitigt werden konnte.

Die Aufgabe der vorliegenden Erfindung besteht darin, ein Verfahren zu Hydrierungen von Phthalaten mit Regenerieren des eingesetzten Rutheniumkatalysators zur Verfügung zu stellen. Dieses soll apparativ einfach zu realisieren und in der Durchführung kostengünstig sein. Insbesondere soll sich damit eine mehrfache und vollständige Regenerierung des Katalysators erreichen lassen.

Gelöst wird die vorstehende Aufgabe durch ein integriertes Verfahren zum Hydrieren von Phthalaten, Isophthalaten und Terephthalaten und den entsprechenden Säuren, vorzugsweise Phthalaten in Gegenwart eines Rutheniumkatalysators aufweisend einen Katalysator-Regenerierungsschritt mit folgenden Schritten:
(a) Bereitstellen zumindest eines Phthalats, Isophthalats oder Terephthalats sowie eines Rutheniumkatalysators;
(b) Hydrieren der eingesetzten aromatischen Verbindung durch Kontakt mit Wasserstoff in Gegenwart des Rutheniumkatalysators, bis der Katalysator eine reduzierte Hydrierungsaktivität aufweist,
(c) Regenerierung des Katalysators durch Spülen mit Inertgas,
(d) gegebenenfalls Wiederholung der Schritte (a) bis (c).

Durch diese Reaktivierung werden einerseits höhere Umsätze bedingt durch eine erhöhte Katalysatoraktivität erzielt, andererseits werden durch das erfindungsgemäße Verfahren die Katalysatorstandzeiten im Produktionsbetrieb deutlich verlängert.

Das erfindungsgemäße Verfahren eignet sich insbesondere für Ru-Katalysatoren, die in den Anmeldungen EP-A 0 814 098, EP-A 1 169 285 und DE 102 005 029 200 beschrieben und in den dort offenbarten Verfahren eingesetzt werden. Diese Katalysatoren und Verfahren seien nachstehend aufgeführt.

In der gesamten Anmeldung werden die Gruppen des Periodensystems in der CAS-Version bezeichnet.

### BEVORZUGTE KATALYSATOREN

### EP-A 0 814 098

Die nachfolgend beschriebenen Katalysatoren werden in der vorliegenden Anmeldung als "Katalysatorvariante I" bezeichnet werden.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird.

Die Begriffe *"Makroporen"* und *"Mesoporen"* werden im Rahmen der vorliegenden Erfindung so verwendet, wie sie in Pure Appl. Chem., 45, S. 79 (1976*)* definiert sind, nämlich als Poren, deren Durchmesser oberhalb von 50 nm (Makroporen) oder deren Durchmesser zwischen 2 nm und 50 nm liegt (Mesoporen). *"Mikroporen"* sind ebenfalls entsprechend der obigen Literatur definiert und bezeichnen Poren mit einem Durchmesser von < 2 nm.

Der Gehalt des Aktivmetalls beträgt im Allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators.

Die Metalloberfläche auf der Katalysatorvariante I beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in "Characterization of Heterogeneous Catalysts", Hrsg. Francis Delanney, Marcel Dekker, New York 1984, S. 310 - 324, beschriebenen Chemisorptionsverfahren bestimmt.

In der Katalysatorvariante I beträgt das Verhältnis der Oberflächen des/der Aktivmetalls/- metalle und des Katalysatorträgers vorzugsweise weniger als ungefähr 0,05, wobei der untere Grenzwert bei ungefähr 0,0005 liegt.

Die Katalysatorvariante I umfasst ein Trägermaterial, das makroporös ist und einen mittleren Porendurchmesser von mindestens ungefähr 50 nm, vorzugsweise mindestens ungefähr 100 nm, insbesondere mindestens ungefähr 500 nm aufweist und dessen Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche nach BET des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Entsprechende Katalysatorträger bzw. Verfahren zu deren Herstellung sind offenbart in den folgenden Dokumenten:

Fundamentals of Industrial Catalytic Processes, R. J. Farrauto, C. H. Bartholomew, First Edition 1997, Seiten 16, 17, 57 bis 62, 88 bis 91, 110 bis 111; Oberlander, R. K., 1984 Aluminas for Catalysts, in Applied Industrial Catalysis, e.g. D. E. Leach, Academic Press, Vol. 3, Kapitel 4; US3,245,919; WO 93/04774; EP-A 0 243 894; Ullmann's Encyclopedia of Industrial Chemistry, 5. Ed., Vol. Al, p. 588 bis 590; VCH 1985

### EP-A 1 169 285

Die nachfolgend beschriebenen Katalysatoren werden in der vorliegenden Anmeldung als "Katalysatorvariante II" bezeichnet werden. Von dieser Variante II existieren verschiedene Untervarianten.

### Untervariante 1

Dieser Katalysator entspricht demjenigen, der vorstehend unter EP-A 0 814 089 beschrieben wurde.

Es wird weiterhin beschrieben die erfindungsgemäß verwendete Untervariante 1 a, die eine bevorzugte Ausführungsform der Untervariante 1 darstellt. Die verwendbaren Trägermaterialien sind solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g aufweisen. Bevorzugt liegt der mittlere Porendurchmesser des dort verwendeten Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers. Auch dieser Katalysator weist bzgl. der Porendurchmesserverteilung die bereits oben beschriebene Bimodalität mit den analogen Verteilungen und das entsprechend bevorzugte Porenvolumen auf. Weitere Details bezüglich Untervariante 1a sind der DE-A 196 04 791.9 zu entnehmen, deren Inhalt durch Bezugnahme vollständig in die vorliegende Anmeldung einbezogen wird.

### Untervariante 2

Die Untervariante 2 enthält ein oder mehrere Metalle der VIII. Nebengruppe des Periodensystems als Aktivkomponente(n) auf einem Träger, wie hierin definiert. Bevorzugt wird Ruthenium als Aktivkomponente verwendet.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. Lemaitre et al., "Characterization of Heterogeneous Catalysts", Hrsg. Francis Delanney, Marcel Dekker, New York (1984), S. 310 - 324, beschrieben ist.

In der Untervariante 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die bei der Untervariante 2 verwendbaren Trägermaterialien besitzen Makroporen und Mesoporen.

Dabei weisen die verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30% und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

### DE 102 005 029 200

Die nachfolgend offenbarten Katalysatoren werden in der vorliegenden Anmeldung als Katalysatorvariante III oder auch "Schalenkatalysator" bezeichnet werden.

Gegenstand ist ein Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial.

Dieser Schalen katalysator ist dann **dadurch gekennzeichnet, dass** die Menge des Aktivmetalls < 1 Gew.-%, bevorzugt 0,1 bis 0,5 Gew.-%, besonders bevorzugt 0,25 bis 0,35 Gew.-%, bezogen auf das Gesamtgewicht des Katalysators, beträgt und mindestens 60 Gew.-%, besonders bevorzugt 80 Gew.-% des Aktivmetalls, bezogen auf die Gesamtmenge des Aktivmetalls, in der Schale des Katalysators bis zu einer Eindringtiefe von 200 µm vorliegen. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Weitere Informationen bezüglich den vorstehend genannten Messverfahren und Techniken sind zum Beispiel "Spectroscopy in Catalysis" von J.W. Niemantsverdriet, VCH, 1995 offenbart.

Der Schalenkatalysator zeichnet sich dadurch aus, dass die überwiegende Menge des Aktivmetalls in der Schale bis zu einer Eindringtiefe von 200 µm, also nahe der Oberfläche des Schalenkatalysators vorliegt. Dagegen liegt im Inneren (Kern) des Katalysators keine oder nur eine sehr geringe Menge des Aktivmetalls vor. Überraschenderweise wurde gefunden, dass die Katalysatorvariante III - trotz der geringen Menge an Aktivmetall - eine sehr hohe Aktivität bei der Hydrierung von organischen Verbindungen, die hydrierbare Gruppen enthalten, insbesondere bei der Hydrierung von carbocyclischen aromatischen Gruppen, bei sehr guten Selektivitäten, aufweist. Insbesondere nimmt die Aktivität der Katalysatorvariante III über einen langen Hydrierzeitraum nicht ab.

Ganz besonders bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 bis 200 µm, vor.

Der Schalenkatalysator zeichnet sich in einer weiteren besonders bevorzugten Ausführungsform dadurch aus, dass im (FEG)-TEM (Field Emission Gun -Transmission Electron Microscopy) mit EDXS nur in den äußersten 200 µm, bevorzugt 100 µm, ganz besonders bevorzugt 50 µm (Eindringtiefe) Aktivmetallteilchen nachweisen können. Teilchen kleiner 1 nm können nicht nachgewiesen werden.

Als Aktivmetall kann Ruthenium alleine oder zusammen mit mindestens einem weiteren Metall der Nebengruppen IB, VIIB oder VIII des Periodensystems der Elemente (CAS-Version) eingesetzt werden. Neben Ruthenium geeignete weitere Aktivmetalle sind z.B. Platin, Rhodium, Palladium, Iridium, Cobalt oder Nickel oder ein Gemisch aus zwei oder mehr davon. Unter den ebenfalls verwendbaren Metallen der Nebengruppen IB und/oder VIIB des Periodensystems der Elemente sind z.B. Kupfer und/oder Rhenium geeignet. Bevorzugt wird Ruthenium alleine als Aktivmetall oder zusammen mit Platin oder Iridium in dem Schalenkatalysator eingesetzt; ganz besonders bevorzugt wird Ruthenium alleine als Aktivmetall eingesetzt.

Der Schalenkatalysator zeigt die vorstehend erwähnte sehr hohe Aktivität bei einer geringen Beladung mit Aktivmetall, die < 1 Gew.-%, bezogen auf das Gesamtgewicht des Katalysators, beträgt. Bevorzugt beträgt die Menge des Aktivmetalls in dem erfindungsgemäßen Schalenkatalysator 0,1 bis 0,5 Gew.-%, besonders bevorzugt 0,25 bis 0,35 Gew.-%. Es wurde gefunden, dass die Eindringtiefe des Aktivmetalls in das Trägermaterial von der Beladung der Katalysatorvariante III mit Aktivmetall abhängig ist. Bereits bei einer Beladung der Katalysatorvariante III mit 1 Gew.-% oder mehr, z.B. bei einer Beladung mit 1,5 Gew.-%, ist im Inneren des Katalysators, d.h. in einer Eindringtiefe von 300 bis 1000 µm eine wesentliche Menge Aktivmetall vorhanden, die die Aktivität des Hydrierkatalysators, insbesondere die Aktivität über einen langen Hydrierzeitraum, beeinträchtigt, insbesondere bei schnellen Reaktionen, wobei Wasserstoffmangel im Inneren des Katalysators (Kern) auftreten kann.

Es liegen in dem Schalenkatalysator mindestens 60 Gew.-% des Aktivmetalls, bezogen auf die Gesamtmenge des Aktivmetalls, in der Schale des Katalysators bis zu einer Eindringtiefe von 200 µm vor. Bevorzugt liegen in dem Schalenkatalysator mindestens 80 Gew.-% des Aktivmetalls, bezogen auf die Gesamtmenge des Aktivmetalls, in der Schale des Katalysators bis zu einer Eindringtiefe von 200 µm vor. Ganz besonders bevorzugt ist ein Schalenkatalysator, in dem kein Aktivmetall im Inneren des Katalysators nachgewiesen werden kann, d.h. Aktivmetall liegt nur in der äußersten Schale, zum Beispiel in einer Zone bis zu einer Eindringtiefe von 100 bis 200 µm Zone, vor. In einer weiteren bevorzugten Ausführungsform liegen 60 Gew.-%, bevorzugt 80 Gew.-%, bezogen auf die Gesamtmenge des Aktivmetalls, in der Schale des Katalysators bis zu einer Eindringtiefe von 150 µm vor. Die vorstehend genannten Daten werden mittels SEM (scanning electron microscopy) EPMA (electron probe microanalysis) - EDXS (energy dispersive X-ray spectroscopy) ermittelt und stellen gemittelte Werte dar. Zur Ermittlung der Eindringtiefe der Aktivmetallteilchen werden mehrere Katalysatorteilchen (z.B. 3, 4 oder 5) quer zur Strangachse (wenn der Katalysator in Form von Strängen vorliegt) angeschliffen. Mittels Linescans werden dann die Profile der Aktivmetall/Si Konzentrationsverhältnisse erfasst. Auf jeder Messlinie werden mehrere zum Beispiel 15 bis 20 Messpunkte in gleichen Abständen gemessen; die Messfleckgröße beträgt circa 10 µm *10 µm. Nach Integration der Aktivmetallmenge über die Tiefe kann die Häufigkeit des Aktivmetalls in einer Zone bestimmt werden.

Ganz besonders bevorzugt beträgt die Menge des Aktivmetalls, bezogen auf das Konzentrationsverhältnis von Aktivmetall zu Si, an der Oberfläche des Schalenkatalysators, 2 bis 25 %, bevorzugt 4 bis 10 %, besonders bevorzugt 4 bis 6 %, ermittelt mittels SEM EPMA - EDXS. Die Oberflächeanalyse erfolgt mittels Bereichsanalysen von Bereichen von 800 µm x 2000 µm und mit einer Informationstiefe von circa 2 µm. Die Elementzusammensetzung wird in Gew. % (normiert auf 100 %) bestimmt. Das mittlere Konzentrationsverhältnis (Aktivmetall/Si) wird über 10 Messbereiche gemittelt.

Unter der Oberfläche des Schalenkatalysators ist die äußere Schale des Katalysators bis zu einer Eindringtiefe von circa 2 µm zu verstehen. Diese Eindringtiefe entspricht der Informationstiefe bei der vorstehend erwähnten Oberflächenanalyse.

Ganz besonders bevorzugt ist ein Schalenkatalysator, worin die Menge des Aktivmetalls, bezogen auf das Gewichtsverhältnis von Aktivmetall zu Si (Gew./Gew. in %), an der Oberfläche des Schalenkatalysators 4 bis 6 % beträgt, in einer Eindringtiefe von 50 µm 1,5 bis 3 % und im Bereich von 50 bis 150 µm Eindringtiefe 0,5 bis 2 %, ermittelt mittels SEM EPMA (EDXS), beträgt. Die genannten Werte stellen gemittelte Werte dar.

Des Weiteren nimmt die Größe der Aktivmetallteilchen bevorzugt mit zunehmender Eindringtiefe ab, ermittelt mittels (FEG)-TEM-Analyse.

Das Aktivmetall liegt in dem Schalenkatalysator bevorzugt teilweise oder vollständig kristallin vor. In bevorzugten Fällen kann in der Schale des Schalenkatalysators mittels SAD (Selected Area Diffraction) oder XRD (X-Ray Diffraction) feinstkristallines Aktivmetall nachgewiesen werden.

Der Schalenkatalysator kann zusätzlich Erdalkalimetallionen (M²⁺), also M = Be, Mg, Ca, Sr und/oder Ba, insbesondere Mg und/oder Ca, ganz besonders Mg enthalten. Der Gehalt an Erdalkalimetallion/en (M²⁺) im Katalysator beträgt bevorzugt 0,01 bis 1 Gew.-%, insbesondere 0,05 bis 0,5 Gew.-%, ganz besonders 0,1 bis 0,25 Gew.-%, jeweils bezogen auf das Gewicht des Siliziumdioxid-Trägermaterials.

Ein wesentlicher Bestandteil der Katalysatorvariante III ist das Trägermaterial auf Basis von Siliziumdioxid, im Allgemeinen amorphem Siliziumdioxid. Unter dem Begriff "amorph" versteht man in diesem Zusammenhang, dass der Anteil kristalliner Siliziumdioxid-Phasen weniger als 10 Gew.-% des Trägermaterials ausmacht. Die zur Herstellung der Katalysatoren verwendeten Trägermaterialien können allerdings Überstrukturen aufweisen, die durch regelmäßige Anordnung von Poren im Trägermaterial gebildet werden.

Als Trägermaterialien kommen grundsätzlich amorphe Siliziumdioxid-Typen in Betracht, die wenigstens zu 90 Gew.-% aus Siliziumdioxid bestehen, wobei die verbleibenden 10 Gew.-%, vorzugsweise nicht mehr als 5 Gew.-%, des Trägermaterials auch ein anderes oxidisches Material sein können, z.B. MgO, CaO, TiO₂, ZrO₂, Fe₂O₃ und/oder Alkalimetalloxid.

In einer bevorzugten Ausführungsform der Erfindung ist das Trägermaterial halogenfrei, insbesondere chlorfrei, d. h. der Gehalt an Halogen im Trägermaterial beträgt weniger als 500 Gew.-ppm, z.B. im Bereich von 0 bis 400 Gew.-ppm. Somit ist ein Schalenkatalysator bevorzugt, der weniger als 0,05 Gew.-% Halogenid (ionenchromatographisch bestimmt), bezogen auf das Gesamtgewicht des Katalysators, enthält.

Bevorzugt sind Trägermaterialien, die eine spezifische Oberfläche im Bereich von 30 bis 700 m²/g, vorzugsweise 30 bis 450 m²/g, (BET-Oberfläche nach DIN 66131) aufweisen.

Geeignete amorphe Trägermaterialien auf Basis von Siliziumdioxid sind dem Fachmann geläufig und kommerziell erhältlich (siehe z.B. O.W. Flörke, "Silica" in Ullmann's Encyclopedia of Industrial Chemistry 6th Edition on CD-ROM). Sie können sowohl natürlichen Ursprungs als auch künstlich hergestellt worden sein. Beispiele für geeignete amorphe Trägermaterialien auf Basis von Siliziumdioxid sind Kieselgele, Kieselgur, pyrogene Kieselsäuren und Fällungskieselsäuren. In einer bevorzugten Ausführungsform der Erfindung weisen die Katalysatoren Kieselgele als Trägermaterialien auf.

Je nach Ausgestaltung der Erfindung kann das Trägermaterial unterschiedliche Gestalt aufweisen. Sofern das Verfahren, in dem die Schalenkatalysatoren eingesetzt werden, als Suspensionsverfahren ausgestaltet ist, wird man zur Herstellung der Katalysatoren üblicherweise das Trägermaterial in Form eines feinteiligen Pulvers einsetzen. Vorzugsweise weist das Pulver Teilchengrößen im Bereich von 1 bis 200 µm insbesondere 1 bis 100 µm auf. Bei Einsatz des erfindungsgemäßen Schalenkatalysators in Katalysatorfestbetten verwendet man üblicherweise Formkörper aus dem Trägermaterial, die z.B. durch Extrudieren, Strangpressen oder Tablettieren erhältlich sind und die z.B. die Form von Kugeln, Tabletten, Zylindern, Strängen, Ringen bzw. Hohlzylindern, Sternen und dergleichen aufweisen können. Die Abmessungen dieser Formkörper bewegen sich üblicherweise im Bereich von 0,5 mm bis 25 mm. Häufig werden Katalysatorstränge mit Strangdurchmessern von 1,0 bis 5 mm und Stranglängen von 2 bis 25 mm eingesetzt. Mit kleineren Strängen können im Allgemeinen höhere Aktivitäten erzielt werden; jedoch zeigen diese oft keine ausreichende mechanische Stabilität im Hydrierverfahren. Daher werden ganz besonders bevorzugt Stränge mit Strangdurchmessern im Bereich von 1,5 bis 3 mm eingesetzt.

### BEVORZUGTE VERFAHREN ZUR HYDRIERUNG VON PHTHALATEN UNTER EINSATZ DER KATALYSATOREN

Die vorstehend beschriebenen Katalysatoren (Katalysatorvarianten I, II und III und genannte Untervarianten) werden bevorzugt als Hydrierkatalysator eingesetzt. Sie sind insbesondere zur Hydrierung einer carbocyclischen aromatischen Gruppe zu der entsprechenden carbocyclischen aliphatischen Gruppe. Besonders bevorzugt erfolgt dabei eine vollständige Hydrierung der aromatischen Gruppe. Erfindungsgemäß sind dies Phthalate, wobei unter vollständiger Hydrierung ein Umsatz der zu hydrierenden Verbindung von im Allgemeinen > 98 %, bevorzugt > 99 %, besonders bevorzugt > 99,5 %, ganz besonders bevorzugt > 99,9 %, insbesondere >99,99 % und speziell >99,995 % zu verstehen ist.

Bei einem Einsatz der Katalysatoren I, II und/oder III zur Hydrierung von aromatischen Dicarbonsäureestern, insbesondere Phthalsäureestern zu den entsprechenden Dialkylcyclohexandicarboxylaten werden somit ebenfalls die typischen Spezifikationen, die einen Restgehalt des aromatischen Dicarbonsäureesters, insbesondere Phthalsäureester-Restgehalt, von < 100 ppm fordern (das entspricht einem Umsatz von > 99,99%), eingehalten. Bevorzugt ist, wie erwähnt, der Umsatz bei einer Hydrierung von aromatischen Dicarbonsäureestern, insbesondere Phthalsäureestern, mit dem erfindungsgemäßen Schalenkatalysator > 99,995%.

Ein weiterer Gegenstand der vorliegenden Anmeldung ist daher ein Verfahren zur Hydrierung von Phthalaten zu den entsprechenden Dicyclohexandicarbonsäure-Derivaten.

Die carbocyclische aromatische Gruppe ist bevorzugt Teil eines aromatischen Kohlenwasserstoffs, der die folgende allgemeine Formel aufweist:

(A)-(B)ₙ

worin die Symbole die folgende Bedeutung haben:
- A: Phenylen C₆H₄
- n: 2
- B ist: COOR, wobei R H, Alkyl, substituiertes Alkyl, Cycloalkyl, substituiertes Cyclo- alkyl, Aryl oder substituiertes Aryl bedeutet; bevorzugt R H oder C₁-₂₀-Alkyl be- deutet.

Unter Alkyl sind gemäß der vorliegenden Anmeldung - wenn nicht anders angegeben - verzweigte oder lineare, gesättigte acyclische Kohlenwasserstoff-Reste zu verstehen. Allgemein weisen die Alkyl-Reste 1 bis 20 Kohlenstoffatome auf.

In der oben erwähnten Gruppe COOR bedeutet R H oder verzweigtes oder lineares Alkyl, bevorzugt H oder C₁₋₁₂-Alkyl. Mehr bevorzugt sind C₄₋₁₀-Alkylgruppen, besonders bevorzugt C₈₋₁₀-Alkylgruppen. Diese können verzweigt oder unverzweigt sein und sind bevorzugt verzweigt. Bei den Alkylgruppen mit mehr als drei Kohlenstoffatomen kann es sich um Isomerengemische verschiedener Alkylgruppen mit derselben Kohlenstoffzahl handeln. Ein Beispiel ist eine C₉-Alkylgruppe, wobei es sich um eine Isononylgruppe handeln kann, also um ein Isomerengemisch verschiedener C₉-Alkylgruppen. Gleiches gilt z.B. für eine C₈-Alkylgruppe. Solche Isomerengemische werden ausgehend von den den Alkylgruppen entsprechenden Alkoholen erhalten, die aufgrund ihres - dem Fachmann bekannten - Herstellungsverfahrens als Isomerengemische anfallen.

Unter Cycloalkyl sind gemäß der vorliegenden Anmeldung gesättigte cyclische nichtaromatische Kohlenwasserstoff-Reste zu verstehen, die aus einem einzigen Ring oder mehreren kondensierten Ringen aufgebaut sind. Geeignete Cycloalkyl-Reste sind beispielsweise Cyclopentyl, Cyclohexyl, Cyclooctyl, Bicyclooctyl usw. Bevorzugt weisen die Cycloalkyl-Reste zwischen 3 und 50 Kohlenstoffatome, besonders bevorzugt zwischen 3 und 20 Kohlenstoffatome, ganz besonders bevorzugt zwischen 3 und 8 Kohlenstoffatome und insbesondere zwischen 3 und 6 Kohlenstoffatome auf.

Substituierte Cycloalkyl-Reste sind solche, in denen ein oder mehrere Wasserstoffatome eines beliebigen Kohlenstoffatoms des Kohlenstoffrings durch eine andere Gruppe ersetzt sind. Solche andere Gruppen sind beispielsweise Halogen, Alkyl, Alkenyl, Alkinyl, substituiertes Alkyl, substituiertes Alkenyl, substituiertes Alkinyl, Aryl, substituiertes Aryl, Cycloalkyl, Cycloalkenyl, substituiertes Cycloalkyl, substituiertes Cycloalkenyl, ein aliphatischer heterocyclischer Rest, ein substituierter aliphatischer heterocyclischer Rest, Heteroaryl, substituiertes Heteroaryl, Alkoxy, Aryloxy, Boryl, Phosphino, Amino, Silyl, und Kombinationen davon. Beispiele für substituierte Cycloalkyl und Cycloalkenyl-Reste sind 4-Dimethylaminocyclohexyl, 4,5-Dibromocyclohept-4-enyl u. a.

In einer bevorzugten Ausführungsform des Hydrierverfahrens ist der aromatische Kohlenwasserstoff ausgewählt aus der Gruppe bestehend aus Phthalsäure (Benzol-1,2-dicarbonsäure) und deren Isomeren Benzol-1,3-dicarbonsäure (Isophthalsäure) und Benzol-1,4-dicarbonsäure (Terephthalsäure) und C₁-C₂₀-Alkylestern der Phthalsäure, Isophthalsäure und Terephthalsäure, vorzugsweise C₁₋₁₂-Alkylestern der Phthalsäure, Isophthalsäure und Terephthalsäure, wobei die C₁₋₁₂-Alkylreste linear oder verzweigt sein können, z.B. Dimethylphthalat, Dimethylisophthalat, Dimethylterephthalat, Di-2-propylheptylphthalat, Di-2-propylheptylisophthalat, Di-2-propylheptylterephthalat, Di-2-ethylhexylphthalat, Di-2-ethylhexylisophthalat, Di-2-ethylhexylterephthalat, Dioctylphthalat, Dioctylisophthalat, Dioctylterephthalat, Diisononylphthalat, Diisononylisophthalat, Diisononylterephthalat. In dem erfindungsgemäßen Verfahren werden somit bevorzugt aromatische Carbonsäuren wie Phthalsäure, Isophthalsäure und Terephthalsäure zu cycloaliphatischen Carbonsäuren so wie die entsprechenden C₁₋₁₂-Alkylester der Phthalsäure, Isophthalsäure und Terephthalsäure zu den entsprechenden aliphatischen Carbonsäure(estern) hydriert, zum Beispiel Dimethylphthalat zu Dimethylcyclohexandicarboxylat, Di-2-propylheptylphthalat zu Di-2-propylheptylcyclohexandicarboxylat, Di-2-ethylhexylphthalat zu Di-2-ethylhexyl-cyclohexandicarboxylat, Dioctylphthalat zu Dioctylcyclohexandicarboxylat und Diisononylphthalat zu Diisononylcyclohexandicarboxylat, hydriert. Insbesondere sind im Rahmen der vorliegenden Erfindung Phthalate bevorzugt.

Nachfolgend werden die erfindungsgemäßen Phthalate spezifiziert.

Bei der Herstellung von Diisononylphthalat stammen die Isononylreste aus einer an sich bekannten Veresterungsreaktion mit Isononanolen. Hierbei werden bevorzugt Isononanole (C9-Alkohole) eingesetzt, die Verzweigungsgrade (ISO-Index) von im Allgemeinen 0,10 bis 4, bevorzugt 0,5 bis 3, besonders bevorzugt 0,8 bis 2 und insbesondere bei 1 bis 1,5 aufweisen, d.h. im Allgemeinen handelt es sich bei den jeweiligen Alkoholen um Gemische verschiedener Isomere. Ganz besonders bevorzugt werden C9-Alkoholgemische mit einem ISO-Index vom 1 bis 1,5, insbesondere Nonanolgemische mit einem ISO-Index von 1,25 bzw. 1,6 eingesetzt.

Der ISO-Index ist eine dimensionslose Größe, die mittels Gaschromatographie bestimmt wird.

Er berechnet sich aus dem Verzweigungsgrad der in dem Alkoholgemisch enthaltenen Komponenten und der Menge der entsprechenden Komponenten (ermittelt mittels Gaschromatographie). Die genaue Berechnungsmethode ist dem Fachmann bekannt.

Die Isononanole werden gemäß dem Fachmann bekannten Verfahren hergestellt. Es wird besonders bevorzugt ein Nonanol-Gemisch eingesetzt, worin 0 bis 20 Gew.-%, bevorzugt 0,5 bis 18 Gew.-%, besonders bevorzugt 6 bis 16 Gew.-% des Nonanol-Gemisches keine Verzweigung aufweisen, 5 bis 90 Gew.-%, bevorzugt 10 bis 80 Gew.-%, besonders bevorzugt 45 bis 75 Gew.-%, eine Verzweigung, 5 bis 70 Gew.-%, bevorzugt 10 bis 60 Gew.-%, besonders bevorzugt 15 bis 35 Gew.-% zwei Verzweigungen, 0 bis 10 Gew.-%, bevorzugt 0 bis 8 Gew.-%, besonders bevorzugt 0 bis 4 Gew.-% drei Verzweigungen aufweisen und 0 bis 40 Gew.-%, bevorzugt 0,1 bis 30 Gew.-%, besonders bevorzugt 0,5 bis 6,5 Gew.-%. sonstige Komponenten sind. Unter sonstigen Komponenten sind im Allgemeinen Nonanole mit mehr als drei Verzweigungen, Decanole oder Octanole zu verstehen, wobei die Summe der genannten Komponenten 100 Gew.-% ergibt.

Eine besonders bevorzugte Ausführungsform eines Nonanol-Gemisches, das zur Herstellung von bevorzugt verwendeten Cyclohexanpolycarbonsäurederivaten eingesetzt wird, weist die folgende Zusammensetzung auf:
- 1,73 bis 3,73 Gew.-%, bevorzugt 1,93 bis 3,53 Gew.-%, besonders bevorzugt 2,23 bis 3,23 Gew.-% 3-Ethyl-6-methyl-hexanol;
- 0,38 bis 1,38 Gew.-%, bevorzugt 0,48 bis 1,28 Gew.-%, besonders bevorzugt 0,58 bis 1,18 Gew.-% 2,6 Dimethylheptanol;
- 2,78 bis 4,78 Gew.-%, bevorzugt 2,98 bis 4,58 Gew.-%, besonders bevorzugt 3,28 bis 4,28 Gew.-% 3,5-Dimethylheptanol;
- 6,30 bis 16,30 Gew.-%, bevorzugt 7,30 bis 15,30 Gew.-%, besonders bevorzugt 8,30 bis 14,30 Gew.-% 3,6-Dimethylheptanol;
- 5,74 bis 11,74 Gew.-%, bevorzugt 6,24 bis 11,24 Gew.-%, besonders bevorzugt 6,74 bis 10,74 Gew.-% 4,6-Dimethylheptanol;
- 1,64 bis 3,64 Gew.-%, bevorzugt 1,84 bis 3,44 Gew.-%, besonders bevorzugt 2,14 bis 3,14 Gew.-% 3,4,5-Trimethylhexanol;
- 1,47 bis 5,47 Gew.-%, bevorzugt 1,97 bis 4,97 Gew.-%, besonders bevorzugt 2,47 bis 4,47 Gew.-% 3,4,5-Trimethylhexanol, 3-Methyl-4-ethylhexanol und 3-Ethyl-4-methylhexanol;
- 4,00 bis 10,00 Gew.-%, bevorzugt 4,50 bis 9,50 Gew.-%, besonders bevorzugt 5,00 bis 9,00 Gew.-% 3,4-Dimethylheptanol;
- 0,99 bis 2,99 Gew.-%, bevorzugt 1,19 bis 2,79 Gew.-%, besonders bevorzugt 1,49 bis 2,49 Gew.-% 4-Ethyl-5-methylhexanol und 3-Ethylheptanol;
- 2,45 bis 8,45 Gew.-%, bevorzugt 2,95 bis 7,95 Gew.-%, besonders bevorzugt 3,45 bis 7,45 Gew.-% 4,5-Dimethylheptanol und 3-Methyloctanol;
- 1,21 bis 5,21 Gew.-%, bevorzugt 1,71 bis 4,71 Gew.-%, besonders bevorzugt 2,21 bis 4,21 Gew.-% 4,5-Dimethylheptanol;
- 1,55 bis 5,55 Gew.-%, bevorzugt 2,05 bis 5,05 Gew.-%, besonders bevorzugt 2,55 bis 4,55 Gew.-% 5,6-Dimethylheptanol;
- 1,63 bis 3,63 Gew.-%, bevorzugt 1,83 bis 3,43 Gew.-%, besonders bevorzugt 2,13 bis 3,13 Gew.-% 4-Methyloctanol;
- 0,98 bis 2,98 Gew.-%, bevorzugt 1,18 bis 2,78 Gew.-%, besonders bevorzugt 1,48 bis 2,48 Gew.-% 5-Methyloctaonol;
- 0,70 bis 2,70 Gew.-%, bevorzugt 0,90 bis 2,50 Gew.-%, besonders bevorzugt 1,20 bis 2,20 Gew.-% 3,6,6-Trimethylhexanol;
- 1,96 bis 3,96 Gew.-%, bevorzugt 2,16 bis 3,76 Gew.-%, besonders bevorzugt 2,46 bis 3,46 Gew.-% 7-Methyloctanol;
- 1,24 bis 3,24 Gew.-%, bevorzugt 1,44 bis 3,04 Gew.-%, besonders bevorzugt 1,74 bis 2,74 Gew.-% 6-Methyloctanol;
- 0,1 bis 3 Gew.-%, bevorzugt 0,2 bis 2 Gew.-%, besonders bevorzugt 0,3 bis 1 Gew.-% n-Nonanol;
- 25 bis 35 Gew.-%, bevorzugt 28 bis 33 Gew.-%, besonders bevorzugt 29 bis 32 Gew.-% sonstige Alkohole mit 9 und 10 Kohlenstoffatomen; wobei die Gesamtsumme der genannten Komponenten 100 Gew.-% ergibt.

Eine weitere besonders bevorzugte Ausführungsform eines Nonanol-Gemisches, das zur Herstellung von bevorzugt verwendeten Cyclohexanpolycarbonsäurederivaten eingesetzt wird, weist die folgende Zusammensetzung auf:
- 6,0 bis 16,0 Gew.-%, bevorzugt 7,0 bis 15,0 Gew.-%, besonders bevorzugt 8,0 bis 14,0 Gew.-% n-Nonanol;
- 12,8 bis 28,8 Gew.-%, bevorzugt 14,8 bis 26,8 Gew.-%, besonders bevorzugt 15,8 bis 25,8 Gew.-% 6-Methyloctanol;
- 12,5 bis 28,8 Gew.-%, bevorzugt 14,5 bis 26,5 Gew.-%, besonders bevorzugt 15,5 bis 25,5 Gew.-% 4-Methyloctanol;
- 3,3 bis 7,3 Gew.-%, bevorzugt 3,8 bis 6,8 Gew.-%, besonders bevorzugt 4,3 bis 6,3 Gew.-% 2-Methyloctanol;
- 5,7 bis 11,7 Gew.-%, bevorzugt 6,3 bis 11,3 Gew.-%, besonders bevorzugt 6,7 bis 10,7 Gew.-% 3-Ethylheptanol;
- 1,9 bis 3,9 Gew.-%, bevorzugt 2,1 bis 3,7 Gew.-%, besonders bevorzugt 2,4 bis 3,4 Gew.-% 2-Ethylheptanol;
- 1,7 bis 3,7 Gew.-%, bevorzugt 1,9 bis 3,5 Gew.-%, besonders bevorzugt 2,2 bis 3,2 Gew.-% 2-Propylhexanol;
- 3,2 bis 9,2 Gew.-%, bevorzugt 3,7 bis 8,7 Gew.-%, besonders bevorzugt 4,2 bis 8,2 Gew.-% 3,5-Dimethylheptanol;
- 6,0 bis 16,0 Gew.-%, bevorzugt 7,0 bis 15,0 Gew.-%, besonders bevorzugt 8,0 bis 14,0 Gew.-% 2,5-Dimethylheptanol;
- 1,8 bis 3,8 Gew.-%, bevorzugt 2,0 bis 3,6 Gew.-%, besonders bevorzugt 2,3 bis 3,3 Gew.-% 2,3-Dimethylheptanol;
- 0,6 bis 2,6 Gew.-%, bevorzugt 0,8 bis 2,4 Gew.-%, besonders bevorzugt 1,1 bis 2,1 Gew.-% 3-Ethyl-4-methylhexanol;
- 2,0 bis 4,0 Gew.-%, bevorzugt 2,2 bis 3,8 Gew.-%, besonders bevorzugt 2,5 bis 3,5 Gew.-% 2-Ethyl-4-methylhexanol;
- 0,5 bis 6,5 Gew.-%, bevorzugt 1,5 bis 6 Gew.-%, besonders bevorzugt 1,5 bis 5,5 Gew.-% sonstige Alkohole mit 9 Kohlenstoffatomen;
wobei die Gesamtsumme der genannten Komponenten 100 Gew.-% ergibt.

Im Rahmen der vorliegenden Erfindung umfasst der Begriff "Phthalate", "Isophthalate" und "Terephthalate" sowohl die freien Säuren wie auch die genannten Ester.

Das Hydrierverfahren kann in der Flüssigphase oder in der Gasphase durchgeführt werden. Bevorzugt wird das erfindungsgemäße Hydrierverfahren in der Flüssigphase durchgeführt.

Das Hydrierverfahren kann in Abwesenheit eines Lösungs- oder Verdünnungsmittels oder in Gegenwart eines Lösungs- oder Verdünnungsmittels durchgeführt werden, d.h. es ist nicht erforderlich, die Hydrierung in Lösung durchzuführen.

Als Lösungs- oder Verdünnungsmittel kann jedes geeignete Lösungsmittel- oder Verdünnungsmittel eingesetzt werden. Als Lösungs- oder Verdünnungsmittel kommen grundsätzlich solche in Betracht, die die zu hydrierende organische Verbindung möglichst vollständig zu lösen vermögen oder sich mit dieser vollständig mischen und die unter den Hydrierungsbedingungen inert sind, d.h. nicht hydriert werden.

Beispiele für geeignete Lösungsmittel sind cyclische und acyclische Ether, z.B. Tetrahydrofuran, Dioxan, Methyl-tert.-butylether, Dimethoxyethan, Dimethoxypropan, Dimethyldiethylenglykol, aliphatische Alkohole wie Methanol, Ethanol, n- oder Isopropanol, n-, 2-, iso- oder tert.-Butanol, Carbonsäureester wie Essigsäuremethylester, Essigsäureethylester, Essigsäurepropylester oder Essigsäurebutylester, sowie aliphatische Etheralkohole wie Methoxypropanol und cycloaliphatische Verbindungen wie Cyclohexan, Methylcyclohexan und Dimethylcyclohexan.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 3 bis 70 gew.-%igen Lösung der zur Hydrierung vorgesehenen organischen Verbindung führen. Der Einsatz eines Verdünnungsmittels ist vorteilhaft, um eine zu starke Wärmetönung im Hydrierverfahren zu vermeiden. Eine zu starke Wärmetönung kann zu einer Deaktivierung des Katalysators führen und ist daher unerwünscht. Daher ist in dem Hydrierverfahren eine sorgfältige Temperaturkontrolle sinnvoll. Geeignete Hydriertemperaturen sind nachstehend genannt.

Bei Verwendung eines Lösungsmittels wird im Rahmen des erfindungsgemäßen Verfahrens besonders bevorzugt das bei der Hydrierung gebildete Produkt, also bevorzugt der oder die jeweilige(n) Cycloaliphat(en) als Lösungsmittel eingesetzt, gegebenenfalls neben anderen Lösungs- oder Verdünnungsmitteln. In jedem Fall kann ein Teil des im Verfahren gebildeten Produkts dem noch zu hydrierenden Aromaten beigemischt werden. Bei der Hydrierung von Phthalaten werden bevorzugt die entsprechenden Dialkylcyclohexandicarbonsäureester als Lösungsmittel eingesetzt.

Bezogen auf das Gewicht des zur Hydrierung vorgesehenen Phthalate, Isophthalate und Terephthalate wird vorzugsweise die 1 bis 30-fache, besonders bevorzugt die 5 bis 20-fache, insbesondere die 5 bis 10-fache Menge an dem dabei zu bildenden Produkt als Lösungs- oder Verdünnungsmittel zugemischt.

Die eigentliche Hydrierung erfolgt üblicherweise derart, dass die organische Verbindung als flüssige Phase oder Gasphase, bevorzugt als flüssige Phase, mit dem Katalysator in Gegenwart von Wasserstoff in Kontakt gebracht. Die flüssige Phase kann über eine Katalysator-Suspension (Suspensions-Fahrweise) oder ein Katalysator-Festbett (Festbettfahrweise) geleitet werden.

Die Hydrierung kann sowohl kontinuierlich als auch diskontinuierlich ausgestaltet werden, wobei die kontinuierliche Verfahrensdurchführung bevorzugt ist. Vorzugsweise führt man das Verfahren in Rieselreaktoren oder in gefluteter Fahrweise nach der Festbettfahrweise durch. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung nach der Hydrierung am Katalysatorfließbett und am Katalysatorfestbett sind aus dem Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM.

Die Hydrierung kann sowohl bei Wasserstoffnormaldruck als auch bei erhöhtem Wasserstoffdruck, z.B. bei einem Wasserstoffabsolutdruck von wenigstens 1,1 bar, vorzugsweise wenigstens 2 bar durchgeführt werden. Im Allgemeinen wird der Wasserstoffabsolutdruck einen Wert von 325 bar und vorzugsweise 300 bar nicht überschreiten. Bevorzugt liegt der Wasserstoffabsolutdruck im Bereich von 1,1 bis 300 bar.

Die Reaktionstemperaturen betragen im erfindungsgemäßen Verfahren im Allgemeinen wenigstens 30°C und werden häufig einen Wert von 250 °C nicht überschreiten. Bevorzugt führt man das Hydrierverfahren bei Temperaturen im Bereich von 50 bis 200 °C, besonders bevorzugt 70 bis 180°C, und ganz besonders bevorzugt im Bereich von 80 bis 160°C durch. Meist bevorzugt erfolgt die Hydrierung von Phthalaten zum Beispiel bei Temperaturen im Bereich von 75°C bis 170°C, insbesondere 80°C bis 160°C. Als Reaktionsgase kommen neben Wasserstoff auch wasserstoffhaltige Gase in Betracht, die keine Katalysatorgifte wie Kohlenmonoxid oder schwefelhaltige Gase wie H₂S oder COS enthalten, z.B. Mischungen von Wasserstoff mit Inertgasen wie Stickstoff oder Reformer-Abgase, die üblicherweise noch flüchtige Kohlenwasserstoffe enthalten. Bevorzugt setzt man reinen Wasserstoff (Reinheit ≥ 99,9 Vol.-%, besonders ≥ 99,95 Vol.-%, insbesondere ≥ 99,99 Vol.-%) ein.

Aufgrund der hohen Katalysatoraktivität benötigt man vergleichsweise geringe Mengen an Katalysator bezogen auf das eingesetzte Edukt. So wird man bei der diskontinuierlichen Suspensionsfahrweise bevorzugt weniger als 5 mol-%, z.B. 0,2 Mol-% bis 2 Mol-% Aktivmetall, bezogen auf 1 Mol Edukt, einsetzen. Bei kontinuierlicher Ausgestaltung des Hydrierverfahrens wird man üblicherweise das zu hydrierende Edukt mit einer Belastung von 0,05 bis 3 kg/(I(Katalysator)•h), insbesondere 0,15 bis 2 kg/(I(Katalysator)•h), über den Katalysator führen.

### INSBESONDERS BEVORZUGTE HYDRIERVERFAHREN

Die erfindungsgemäße Hydrierung von Phthalaten, Isophthalaten und Terephthalaten, vorzugsweise Phthalaten, umfassend einen Regenerierungsgehalt erfolgt im Allgemeinen bei einer Temperatur von 50°C bis 250°C, bevorzugt 70°C bis 220°C. Der Druck beträgt im Allgemeinen ≥ 10 bar.

Vorzugsweise wird im Rahmen des vorliegenden Verfahrens Diisononylphthalat bei einem Druck im Bereich von etwa 200 bis etwa 250 bar zu Diisononylcyclohexandicarboxylat hydriert.

Die Hydrierung kann im Allgemeinen in der Suspensions- oder Festbettfahrweise durchgeführt werden, wobei eine Durchführung in der Festbettfahrweise bevorzugt ist. Besonders bevorzugt wird das Hydrierverfahren mit Flüssigkeitsumlauf durchgeführt, wobei die Hydrierwärme über einen Wärmetauscher abgeführt und genutzt werden kann. Das Zulauf/Kreislauf-Verhältnis beträgt bei einer Durchführung des Hydrierverfahrens mit Flüssigkeitsumlauf von im Allgemeinen 1:5 bis 1:40, bevorzugt von 1:10 bis 1:30.

Um einen vollständigen Umsatz zu erzielen, kann eine Nachreaktion des Hydrieraustrags erfolgen. Dazu kann der Hydrieraustrag im Anschluss an das Hydrierverfahren in der Gasphase oder in der Flüssigphase im geraden Durchgang, durch einen nachgeschalteten Reaktor geleitet werden. Der Reaktor kann bei Flüssigphasenhydrierung in Rieselfahrweise betrieben oder geflutet betrieben werden. Der Reaktor ist mit dem erfindungsgemäßen oder mit einem anderen, dem Fachmann bekannten, Katalysator befüllt.

### REGENERIERUNGSSCHRITT

Bei Hydrierverfahren, in denen die oben dargestellten Katalysatoren eingesetzt werden, ist nach einer gewissen Katalysatorstandzeit eine Desaktivierung zu beobachten. Ein solcher deaktivierter Rutheniumkatalysator kann durch Spülen in den Zustand der ursprünglichen Aktivität zurückgeführt werden. Die Aktivität lässt sich bis auf > 90 %, vorzugsweise > 95 %, mehr bevorzugt > 98 %, insbesondere > 99 %, meist bevorzugt > 99,5 % des Ursprungswertes wieder herstellen. Die Desaktivierung wird auf Spuren beziehungsweise Reste an dem Katalysator adsorbierten Wasser zurückgeführt. Dies lässt sich überraschenderweise durch das Spülen mit Inertgas rückgängig machen. Der erfindungsgemäße Regenerierungsschritt lässt sich somit auch als Trocknung des Katalysators oder Entfernen von Wasser von diesem bezeichnen.

Spülen bedeutet, dass der Katalysator mit Inertgas in Kontakt gebracht wird. Normalerweise wird dann durch geeignete, dem Fachmann bekannte konstruktive Maßnahmen das Inertgas über den Katalysator geleitet.

Das Spülen mit Inertgas wird bei einer Temperatur von ungefähr 10 bis 350 °C, bevorzugt von ungefähr 50 bis 250 °C, besonders bevorzugt von ungefähr 70 bis 180 °C, am meisten bevorzugt von ungefähr 80 bis 130 °C durchgeführt.

Die beim Spülen angelegten Drücke betragen 0,5 bis 5 bar, vorzugsweise 0,8 bis 2 bar, insbesondere 0,9 bis 1,5 bar.

Erfindungsgemäß wird die Behandlung des Katalysators vorzugsweise mit einem Inertgas durchgeführt. Bevorzugte Inertgase umfassen Stickstoff, Kohlendioxid, Helium, Argon, Neon und Mischungen daraus. Am meisten bevorzugt ist Stickstoff.

Gemäß einer besonderen Ausführungsform der Erfindung wird der erfindungsgemäße Regenerierungsschritt ohne Ausbau des Katalysators in demselben Reaktor durchgeführt, in dem die Hydrierung stattgefunden hat. In besonders vorteilhafter Weise wird das Spülen des Katalysators gemäß der vorliegenden Erfindung bei Temperaturen und Drücken im Reaktor durchgeführt, die entsprechend oder ähnlich der Hydrierungsreaktion sind, wodurch eine nur sehr kurze Unterbrechung des Reaktionsprozesses resultiert.

Gemäß der vorliegenden Erfindung wird das Spülen mit Inertgas mit einem Volumenstrom von 20 bis 200 Nl/h, bevorzugt mit einem Volumenstrom von 50 bis 200 NI/h pro Liter Katalysator durchgeführt.

Das Spülen mit Inertgas wird bevorzugt über eine Zeitdauer von 10 bis 50 Stunden, besonders bevorzugt von 10 bis 20 Stunden, durchgeführt. Beispielsweise beträgt die errechnete Trockenzeit des Katalysatorbettes einer großtechnischen Produktionsanlage mit einer angenommenen Feuchte von 2 beziehungsweise 5 Gew.-% näherungsweise 18 beziehungsweise 30 Stunden. Das Spülen kann in dem erfindungsgemäßen Verfahren sowohl in abwärts gerichteter Richtung (down-flow) als auch in aufwärts gerichteter Richtung (up-flow) durchgeführt werden.

Gegenstand der vorliegenden Erfindung ist ein integriertes Verfahren zum Hydrieren von Phthalaten, Isophthalaten und Terephthalaten und den entsprechenden Säuren, vorzugsweise Phthalaten in Gegenwart eines Rutheniumkatalysators aufweisend einen Katalysator-Regenerierungsschritt mit folgenden Schritten:
(a) Bereitstellen zumindest eines Phthalats, Isophthalats oder Terephthalats sowie eines Rutheniumkatalysators;
(b) Hydrieren der eingesetzten aromatischen Verbindung durch Kontakt mit Wasserstoff in Gegenwart des Rutheniumkatalysators, bis der Katalysator eine reduzierte Hydrierungsaktivität aufweist,
(c) Regenerierung des Katalysators durch Spülen mit Inertgas,
(d) gegebenenfalls Wiederholung der Schritte (a) bis (c).

Der erfindungsgemäße Wasserstoff enthält vorzugsweise keine schädlichen Katalysatorgifte, wie beispielsweise CO. Beispielsweise können Reformergase verwendet werden. Vorzugsweise wird reiner Wasserstoff als Hydriergas verwendet.

Der erfindungsgemäße Regenerierungsschritt eignet sich weiterhin zur Trocknung von Katalysatoren, die während verschiedener Vorgänge wie Wartung oder Lagerung Wasser aufgenommen haben.

Demgemäß ist der Regenerierungsschritt ein Verfahren zur Trocknung und/oder Reaktivierung und/oder Regenerierung eines Katalysators enthaltend Ruthenium auf einem Trägermaterial, bei dem der Katalysator durch Behandlung mit einem Inertgas behandelt wird.

Nach dieser Behandlung besitzt der Katalysator eine höhere katalytische Aktivität als vorher.

Die Erfindung soll anhand der folgenden Beispiele näher erläutert werden.

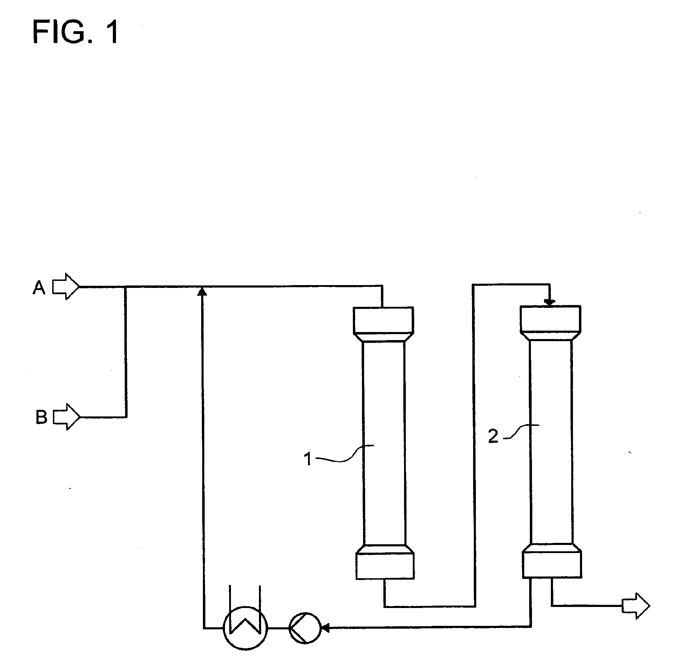

Die Figur 1 zeigt ein Fließbild der Herstellung von 1,2-Diisononylcyclohexandicarbonsäureester (siehe Beispiel 2)

### Herstellungsbeispiel des Rutheniumkatalysators

Ein meso-/makroporöser Aluminiumoxidträger in Form von 3 bis 5 mm-Kugeln mit einem Gesamtvolumen von 0,44 cm³/g, wobei 0,09 cm³/g (20% des Gesamtporenvolumens) von Poren mit einem Durchmesser im Bereich von 50 nm bis 10.000 nm und 0,35 cm³/g (80% des Gesamtporenvolumens) von Poren mit einem Durchmesser im Bereich von 2 nm bis 50 nm gebildet werden, einem mittleren Porendurchmesser im Bereich von 11 nm und einer Oberfläche von 286 m²/g wurde mit einer wässrigen Ruthenium-(III)-nitrat-Lösung getränkt. Das während des Tränkens aufgenommene Lösungsvolumen entsprach dabei in etwa dem Porenvolumen des verwendeten Trägers. Anschließend wurde der mir der Ruthenium-(III)-nitrat-Lösung getränkte Träger bei 120°C getrocknet und bei 200°C im Wasserstoffstrom aktiviert (reduziert). Der so hergestellte Katalysator enthielt 0,5 Gew.-% Ruthenium, bezogen auf das Gewicht des Katalysators. Die Rutheniumoberfläche betrug 0,72 m²/g, das Verhältnis von Ruthenium- zu Trägeroberfläche lag bei 0,0027.

### Beispiel 1 Sorptionsuntersuchungen

Durch Sorptionsmessungen von Wasserdampf an dem wie vorstehend beschrieben hergestellten Katalysator (0,5 % Ru/γ-Al₂O₃), wurde die Affinität des Katalysators zum Wasser ermittelt.

Es zeigte sich, dass der Katalysator schon bei geringen relativen Dampfdrücken von 30% eine Wassermenge von 5% sorbiert. Falls im Reaktor beziehungsweise in den Einsatzstoffen Wasser auch nur in Spuren vorhanden ist, kann dieses Wasser am Katalysator sorbiert werden.

### Beispiel 2 (Vergleich mit und ohne Trocknung)

Bei der Hydrierung von Diisononylphthalat zu DINCH wird festgestellt, dass nach 3 Monaten Betriebszeit der Restphthalatgehalt < 100 ppm nur noch durch sukzessive Reduzierung der Katalysatorbelastung (= Reduzierung der Zufuhr von DINP) erreicht wird. Temperatur und Druckerhöhung führen zu keiner Verbesserung. Die Versuchsanlage ist im Folgenden beschrieben:

Die Anlage besteht aus den zwei hintereinandergeschalteten Reaktoren 1 und 2 (Figur 1). Die Reaktionswärme wird durch Kühlung aus dem Wälzkreis des Reaktors 1 abgezogen. Beide Reaktoren 1 und 2 (in der Figur abgekürzt mit 1 und 2) werden in Rieselfahrweise betrieben. A ist die Zufuhr von DINP, B die Zufuhr von H₂.

In folgender Tabelle 1 sind Reaktorgröße und Katalysatorvolumen zusammengestellt.

| | **Reaktor 1** | **Reaktor 2** |
|---|---|---|
| Katalysatorvolumen | 2,7 l | 0,34 l |
| Reaktordimensionen | 45 x 2000 mm | 22 x 1500 mm |
| | | |
| Höhe der aktiven Schüttung | 1,86 m | 1,14 m |

Zur Trocknung wird die Flüssigkeit aus den Reaktoren 1 und 2 entleert und 7 Tage lang 13 Nm3/h auf 110°C erhitzter Stickstoff über die beiden Reaktoren geleitet. Dadurch wird das auf dem Katalysator adsorbierte Wasser entfernt. Nach der erneuten Inbetriebnahme ist die Aktivität des Katalysators deutlich erhöht. Die produzierte Menge kann verdoppelt werden, der Restphthalatgehalt liegt < 100 ppm. Die Versuche nach 3 Monaten Betriebszeit vor der Trocknung und nach der Trocknung sind in Tabelle 2 zusammengestellt.

**Tabelle 2**

| | Eingang DINP | Temp Reaktor | Druck Reaktor | Temp Reaktor | Druck Reaktor | Produktion DINCH | Rest-aromaten |
|---|---|---|---|---|---|---|---|
| | [kg/h] | 1 [°C] | 1 [bar] | 2 [°C] | 2 [bar] | [kg/h] | [ppm] |
| vor Trocknung | 0,3 | 121 | 264 | 136 | 263 | 0,3 | 95 |
| nach Trocknung | 0,52 | 121 | 265 | 142 | 264 | 0,52 | 50 |

## Patentansprüche

1. Integriertes Verfahren zum Hydrieren von Phthalaten und den entsprechenden Säuren in Gegenwart eines Rutheniumkatalysators mit folgenden Schritten:
(a) Bereitstellen zumindest eines Phthalats, Isophthalats oder Terephthalats sowie eines Rutheniumkatalysators;
(b) Hydrieren der eingesetzten aromatischen Verbindung durch Kontakt mit Wasserstoff in Gegenwart des Rutheniumkatalysators, bis der Katalysator eine reduzierte Hydrierungsaktivität aufweist,
(c) Regenerierung des Katalysators durch Spülen mit Inertgas,
bis zum Erreichen der ursprünglichen Aktivität oder eines Teils der ursprünglichen Aktivität, und
(d) gegebenenfalls Wiederholung der Schritte (a) bis (c).

2. Verfahren nach Anspruch 1, **dadurch gekennzeichnet, dass** das Spülen mit Inertgas bei einer Temperatur von 10 bis 350 C° durchgeführt wird.

3. Verfahren nach Anspruch 1 oder 2, **dadurch gekennzeichnet, dass** der beim Spülen angelegte Druck 0,5 bis 5 bar beträgt.

4. Verfahren nach einem der Ansprüche 1 bis 3, **dadurch gekennzeichnet, dass** das Inertgas ausgewählt ist aus Stickstoff, Kohlendioxid, Helium, Argon, Neon und Mischungen daraus.

5. Verfahren nach einem der Ansprüche 1 bis 4, **dadurch gekennzeichnet, dass** das Spülen mit Inertgas mit einem Volumenstrom von 20 bis 200 NI/h, pro Liter Katalysator durchgeführt wird.

6. Verfahren nach einem der Ansprüche 1 bis 5, **dadurch gekennzeichnet, dass** Spülen mit Inertgas über einen Zeitraum von 10 bis 50 Stunden durchgeführt wird.

7. Verfahren nach einem der Ansprüche 1 bis 6, **dadurch gekennzeichnet, dass** der Regenerierungsschritt durchgeführt wird, bis eine Aktivität von > 90 % des Ursprungswertes erreicht ist.

8. Verfahren nach einem der Ansprüche 1 bis 7, **dadurch gekennzeichnet, dass** der Rutheniumkatalysator ausgewählt ist aus den nachfolgenden Gruppen:
a) Katalysator enthaltend als Aktivmetall Ruthenium alleine oder zusammen mit mindestens einem Metall der I., VII. oder VIII. Nebengruppe des Periodensystems in einer Menge von 0,01 bis 30 Gew.-%, bezogen auf das Gesamtgewicht des Katalysators, aufgebracht auf einem Träger, und wobei 10 bis 50 % des Porenvolumens des Trägers von Makroporen mit einem Porendurchmesser im Bereich von 50 nm bis 10.000 nm und 50 bis 90 % des Porenvolumens des Trägers von Mesoporen mit einem Porendurchmesser im Bereich von 2 bis 50 nm gebildet werden, wobei sich die Summe der Porenvolumina zu 100 % addiert, und
b) Schalenkatalysator enthaltend als Aktivmetall Ruthenium alleine oder zusammen mit mindestens einem weiteren Metall der Nebengruppen IB, VIIB oder VIII des Periodensystems der Elemente (CAS-Version), aufgebracht auf einen Träger enthaltend Siliziumdioxid als Trägermaterial, **dadurch gekennzeichnet, dass** die Menge des Aktivmetalls < 1 Gew.-% beträgt, bezogen auf das Gesamtgewicht des Katalysators, und mindestens 60 Gew.-% des Aktivmetalls in der Schale des Katalysators bis zu einer Eindringtiefe von 200 µm vorliegen, ermittelt mittels SEM-EPMA (EDXS)

9. Verfahren nach Anspruch 8, **dadurch gekennzeichnet, dass** es sich bei dem Katalysator um Katalysator a) handelt und das mindestens eine Metall der I., VII., oder VIII. Nebengruppe des Periodensystems Platin, Kupfer, Rhenium, Kobalt, Nickel oder ein Gemisch aus zwei oder mehr davon ist.

10. Verfahren nach Anspruch 8 oder 9, **dadurch gekennzeichnet, dass** es sich bei dem Katalysator um Katalysator A) handelt und der Träger Aktivkohle, Siliziumcarbid, Aluminiumoxid, Siliciumdioxid, Titanoxid, Zirkoniumoxid, Magnesiumoxid, Zinkoxid oder ein Gemisch aus zwei oder mehr davon ist.

11. Verfahren nach Anspruch 8, **dadurch gekennzeichnet, dass** es sich bei dem Katalysator um Katalysator b) handelt und das mindestens eine Metall der I., VII., oder VIII. Nebengruppe des Periodensystems Platin, Rhodium, Palladium, Iridium, Kobalt, Nickel, ein Gemisch aus zwei oder mehr davon oder Kupfer und/oder Rhenium ist.

12. Verfahren nach einem der Ansprüche 1 bis 11, **dadurch gekennzeichnet, dass** die Phthalate ausgesucht sind aus der Gruppe der Phthalate, Isophthalate und Terephthalate.

13. Verfahren nach Anspruch 12, **dadurch gekennzeichnet, dass** Diisononylphthalat zu Diisononylcyclohexandicarboxylat umgesetzt wird.

## Claims

1. An integrated process for the hydrogenation of phthalates and the corresponding acids in the presence of a ruthenium catalyst, which comprises the following steps:
(a) provision of at least one phthalate, isophthalate or terephthalate and a ruthenium catalyst;
(b) hydrogenation of the aromatic compound used by contact with hydrogen in the presence of the ruthenium catalyst until the catalyst has a reduced hydrogenation activity,
(c) regeneration of the catalyst by flushing with inert gas,
until the original activity or part of the original activity has been attained, and
(d) if appropriate, repetition of the steps (a) to (c).

2. The process according to claim 1, wherein the flushing with inert gas is carried out at a temperature of from 10 to 350°C.

3. The process according to claim 1 or 2, wherein the pressure applied during flushing is from 0.5 to 5 bar.

4. The process according to any of claims 1 to 3, wherein the inert gas is selected from among nitrogen, carbon dioxide, helium, argon, neon and mixtures thereof.

5. The process according to any of claims 1 to 4, wherein the flushing with inert gas is carried out at a volume flow of from 20 to 200 standard l/h per liter of catalyst.

6. The process according to any of claims 1 to 5, wherein flushing with inert gas is carried out for a time of from 10 to 50 hours.

7. The process according to any of claims 1 to 6, wherein the regeneration step is carried out until an activity of > 90% of the original value is attained.

8. The process according to any of claims 1 to 7, wherein the ruthenium catalyst is selected from among the following groups:
a) catalyst comprising, as active metal, ruthenium alone or ruthenium together with at least one metal of transition group I, VII or VIII of the Periodic Table in an amount of from 0.01 to 30% by weight, based on the total weight of the catalyst, applied to a support, wherein from 10 to 50% of the pore volume of the support is formed by macropores having a pore diameter in the range from 50 nm to 10 000 nm and from 50 to 90% of the pore volume of the support being formed by mesopores having a pore diameter in the range from 2 to 50 nm, with the sum of the pore volumes being 100%, and
b) coated catalyst comprising, as active metal, ruthenium alone or ruthenium together with at least one further metal of transition group IB, VIIB or VIII of the Periodic Table of the Elements (CAS version) applied to a support comprising silicon dioxide as support material, wherein the amount of active metal is < 1% by weight, based on the total weight of the catalyst, and at least 60% by weight of the active metal is present in the shell of the catalyst to a penetration depth of 200 µm, determined by means of SEM-EPMA (EDXS).

9. The process according to claim 8, wherein the catalyst is catalyst a) and the at least one metal of transition group I, VII or VIII of the Periodic Table is platinum, copper, rhenium, cobalt, nickel or a mixture of two or more thereof.

10. The process according to claim 8 or 9, wherein the catalyst is catalyst a) and the support is activated carbon, silicon carbide, aluminum oxide, silicon dioxide, titanium oxide, zirconium oxide, magnesium oxide, zinc oxide or a mixture of two or more thereof.

11. The process according to claim 8, wherein the catalyst is catalyst b) and the at least one metal of transition group I, VII or VIII of the Periodic Table is platinum, rhodium, palladium, iridium, cobalt, nickel, a mixture of two or more thereof or copper and/or rhenium.

12. The process according to any of claims 1 to 11, wherein the phthalates are selected from the group consisting of phthalates, isophthalates and terephthalates.

13. The process according to claim 12, wherein diisononyl phthalate is converted into diisononyl cyclohexanedicarboxylate.

## Revendications

1. Procédé intégré d'hydrogénation de phtalates et des acides correspondants en présence d'un catalyseur de ruthénium, comprenant les étapes suivantes :
(a) la préparation d'au moins un phtalate, isophtalate ou téréphtalate, ainsi que d'un catalyseur de ruthénium ;
(b) l'hydrogénation du composé aromatique utilisé par mise en contact avec de l'hydrogène en présence du catalyseur de ruthénium, jusqu'à ce que le catalyseur présente une activité d'hydrogénation réduite,
(c) la régénération du catalyseur par rinçage avec un gaz inerte,
jusqu'à atteindre l'activité initiale ou une partie de l'activité initiale, et
(d) éventuellement la répétition des étapes (a) à (c).

2. Procédé selon la revendication 1, **caractérisé en ce que** le rinçage avec un gaz inerte est réalisé à une température de 10 à 350 °C.

3. Procédé selon la revendication 1 ou 2, **caractérisé en ce que** la pression appliquée pendant le rinçage est de 0,5 à 5 bar.

4. Procédé selon l'une quelconque des revendications 1 à 3, **caractérisé en ce que** le gaz inerte est choisi parmi l'azote, le dioxyde de carbone, l'hélium, l'argon, le néon et leurs mélanges.

5. Procédé selon l'une quelconque des revendications 1 à 4, **caractérisé en ce que** le rinçage avec un gaz inerte est réalisé avec un débit volumique de 20 à 200 Nl/h, par litre de catalyseur.

6. Procédé selon l'une quelconque des revendications 1 à 5, **caractérisé en ce que** le rinçage avec un gaz inerte est réalisé pendant une durée de 10 à 50 heures.

7. Procédé selon l'une quelconque des revendications 1 à 6, **caractérisé en ce que** l'étape de régénération est réalisée jusqu'à atteindre une activité > 90 % de la valeur initiale.

8. Procédé selon l'une quelconque des revendications 1 à 7, **caractérisé en ce que** le catalyseur de ruthénium est choisi parmi les groupes suivants :
a) un catalyseur contenant en tant que métal actif du ruthénium seul ou accompagné d'au moins un métal du groupe de transition I, VII ou VIII du tableau périodique en une quantité de 0,01 à 30 % en poids, par rapport au poids total du catalyseur, appliqué sur un support, 10 à 50 % du volume poreux du support étant formé par des macropores ayant un diamètre de pore dans la plage allant de 50 nm à 10 000 nm et 50 à 90 % du volume poreux du support étant formé par des mésopores ayant un diamètre de pore dans la plage allant de 2 à 50 nm, la somme des volumes poreux étant de 100 %, et
b) un catalyseur à enveloppe contenant en tant que métal actif du ruthénium seul ou accompagné d'au moins un métal supplémentaire des groupes secondaires IB, VIIB ou VIII du tableau périodique des éléments (version CAS), appliqué sur un support contenant du dioxyde de silicium en tant que matériau support, **caractérisé en ce que** la quantité du métal actif est < 1 % en poids, par rapport au poids total du catalyseur, et au moins 60 % en poids du métal actif se trouve dans l'enveloppe du catalyseur jusqu'à une profondeur de pénétration de 200 µm, calculée par SEM-EPMA (EDXS).

9. Procédé selon la revendication 8, **caractérisé en ce que** le catalyseur est un catalyseur a) et le ou les métaux du groupe de transition I, VII ou VIII du tableau périodique sont le platine, le cuivre, le rhénium, le cobalt, le nickel ou un mélange de deux ou plus d'entre eux.

10. Procédé selon la revendication 8 ou 9, **caractérisé en ce que** le catalyseur est un catalyseur a) et le support est du charbon actif, du carbure de silicium, de l'oxyde d'aluminium, du dioxyde de silicium, de l'oxyde de titane, de l'oxyde de zirconium, de l'oxyde de magnésium, de l'oxyde de zinc ou un mélange de deux ou plus d'entre eux.

11. Procédé selon la revendication 8, **caractérisé en ce que** le catalyseur est un catalyseur b) et le ou les métaux du groupe de transition I, VII ou VIII du tableau périodique sont le platine, le rhodium, le palladium, l'iridium, le cobalt, le nickel, un mélange de deux ou plus d'entre eux, ou le cuivre et/ou le rhénium.

12. Procédé selon l'une quelconque des revendications 1 à 11, **caractérisé en ce que** les phtalates sont choisis dans le groupe des phtalates, isophtalates et téréphtalates.

13. Procédé selon la revendication 12, **caractérisé en ce que** le phtalate de diisononyle est transformé en dicarboxylate de diisononylcyclohexane.
